# EUROPEAN PATENT SPECIFICATION

(11) **EP 1 286 625 B1**
(45) Date of publication and mention of the grant of the patent: **22.12.2010**
(21) Application number: 01939887.4
(22) Date of filing: 05.06.2001
(51) Int. Cl.: A61B 18/14

(54) **MULTIPOLAR ELECTRODE SYSTEM FOR RADIOFREQUENCY ABLATION**
VIELFACHPOLARES ELEKTRODENSYSTEM FÜR DIE HOCHFREQUENZABLATION
SYSTEME A ELECTRODE MULTIPOLAIRE POUR ABLATION RADIOELECTRIQUE

(30) Priority: 07.06.2000 US 210103 P
(43) Date of publication of application: 05.03.2003
(73) Proprietor: WISCONSIN ALUMNI RESEARCH FOUNDATION, Madison, WI 53705 (US)
(72) Inventor: MAHVI, David, M., Middleton, WI 53562 (US); LEE, Fred, T., Jr., Madison, WI 53706 (US); WEBSTER, John G., Madison, WI 53705 (US); STAELIN, Stephen, T., Madison, WI 53792 (US); HAMMERICH, Dieter, Madison, WI 53726 (US); TUNGJITKUSOLMUN, Supan, Sathorn, Bangkok 10120 (TH)
(74) Representative: Price, Nigel John King
(86) International application number: PCT/US2001/018088
(87) International publication number: WO 2001/093769

(56) References cited:
- WO-A-00/06046
- US-A- 5 735 847
- US-A- 5 855 576

## Description

The present invention relates to electrodes for radiofrequency ablation of tumors and the like, and in particular to a multipolar electrode system suitable for the ablation of liver tumors.

Ablation of tumors, such as liver (hepatic) tumors, uses heat or cold to kill tumor cells. In cryosurgical ablation, a probe is inserted during an open laparotomy and the tumor is frozen. In radiofrequency ablation (RFA), an electrode is inserted into the tumor and current passing from the electrode into the patient (to an electrical return typically being a large area plate on the patient's skin) destroys the tumor cells through resistive heating.

A simple RFA electrode is a conductive needle having an uninsulated tip placed within the tumor. The needle is energized with respect to a large area contact plate on the patient's skin by an oscillating electrical signal of approximately 460 kHz. Current flowing radially from the tip of the needle produces a spherical or ellipsoidal zone of heating (depending on the length of the exposed needle tip) and ultimately a lesion within a portion of the zone having sufficient temperature to kill the tumor cells. The size of the lesion is limited by fall-off in current density away from the electrode (causing reduced resistive heating), loss of heat to the surrounding tissue, and limits on the amount of energy transferred to the tissue from the electrode. The electrode energy is limited to avoid charring, boiling and vaporization of the tissue next to the electrode, a condition that greatly increases the resistance between the electrode and the remainder of the tumor. The tissue next to the electrode chars first because of the high current densities close to the electrode and thus creates a bottleneck in energy transfer.

Several approaches have been developed to increase energy delivered to tissue without causing charring. A first method places temperature sensors in the tip of the electrode to allow more accurate monitoring of temperatures near the electrode and thereby to allow a closer approach to those energies just short of charring. A second method actively . cools the tip of the electrode with circulated coolant fluids within the electrode itself. A third method increases the area of the electrode using an umbrella-style electrode in which three or more electrode wires extend radially from the tip of the electrode shaft, after it has been positioned in the tumor. The greater surface area of the electrode reduces maximum current densities. The effect of all of these methods is to increase the amount of energy deposited into the tumor and thus to increase the lesion size allowing more reliable ablation of more extensive tumors.

A major advantage of RFA in comparison to cryosurgical ablation is that it may be delivered percutaneously, without an incision, and thus with less trauma to the patient. In some cases, RFA is the only treatment the patient can withstand. Further, RFA can be completed while the patient is undergoing a CAT scan.

Nevertheless, despite the improvements described above, RFA often fails to kill all of the tumor cells and, as a result, tumor recurrence rates of as high as 40% have been reported.

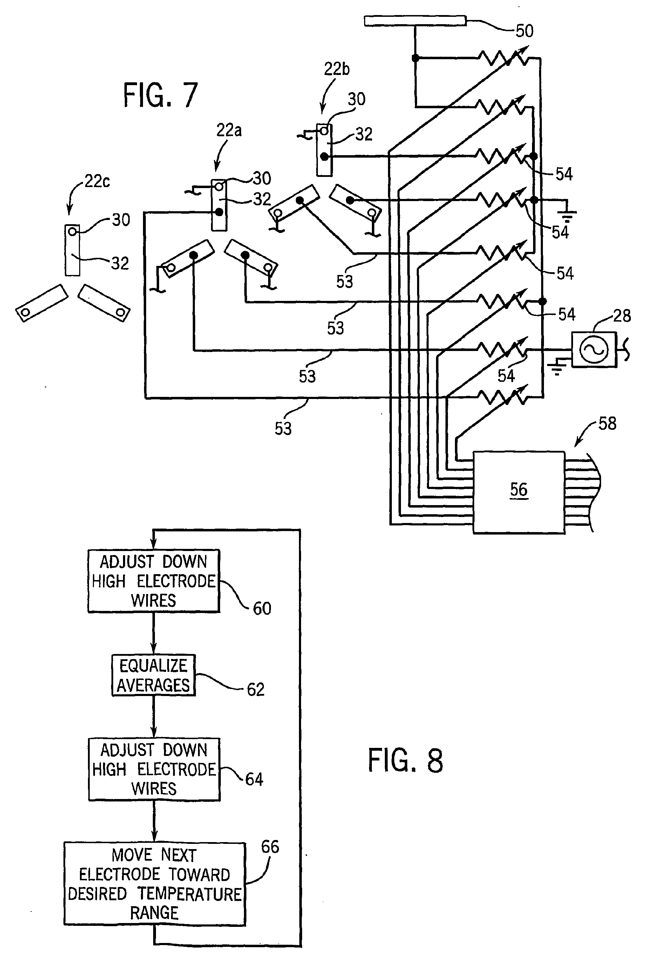

US-A-5 735 847 discloses an ablation apparatus including a multiple antenna device coupled to an electromagnetic energy source that produces an electromagnetic energy ablation output. In the Fig. 8 arrangement, a primary antenna is provided with an insulation sleeve stopping short of the antenna's distal end. At the location of the distal end of the insulation sleeve three secondary antennas are provided extending at angularly offset radial points around the primary antenna. At the distal end of the primary antenna, offset axially along the primary antenna from the first set of secondary antennas, there is provided a pair of further secondary antennas, also positioned at angularly offset radial points around the primary antenna.

According to the present invention there is provided an electrode assembly for ablating tumors in a patient, the assembly comprising:
(a) a support shaft sized for percutaneous placement, said shaft having an outer surface and a distal tip;
(b) first and second wire electrode sets extensible radially from the shaft to an extension radius, the first wire electrode set being positionable at a first location adjacent to a tumour volume and offset axially along the support shaft from the second wire electrode set, which second wire electrode set is positionable at a second location offset from the first location about the tumour volume, the first and second electrode sets each comprising at least three wires positionable at angularly offset radial points around the support shaft;
(c) a power supply connectable between the first and second electrode sets to induce a current flow between the first and second electrode sets whereby to concentrate current induced heating in the tumour volume;
wherein the support shaft has an electrically insulating cover on the outer surface between the first and second locations, said cover extending to the distal tip of the support shaft.

The present inventors have modeled the heating zone of standard RFA electrodes and believe that the high recurrence rate currently associated with RFA may result in part from limitations in the lesion size and irregularities in the lesion shape that can be obtained with these electrodes. Current lesion sizes may be insufficient to encompass the entire volume of a typical hepatic tumor particularly in the presence of nearby blood vessels that act as heat sinks, carrying away energy to reduce the lesion size in their vicinity.

In order to overcome the energy limitations of current electrode designs, the present inventors have adopted a multipolar electrode design that increase lesion size by "focusing" existing energy on the tumor volume between two or more electrodes. By using axially displaced umbrella electrodes supported by an outwardly non-conductive shaft, a more regular lesion area is created than is provided by a single umbrella electrode and the lesion produced is greater in volume than would be obtained by a comparable number of monopolar umbrella electrodes operating individually.

Specifically the electrode assembly of present invention enables the ablation of a tumor in a patient by inserting a first wire electrode set and a second wire electrode set percutaneously at a tumor volume, so that the second wire electrode set is at a second location opposed to and at a predetermined separation from the first wire electrode set at a first location about the tumor volume. First and second electrically isolated wire umbrella electrodes sets extend radially at the first and second locations respectively to an extension radius; and a power supply is connected between the first and second electrode sets to induce a current flow between them through the tumor volume whereby current induced heating is concentrated in the tumor volume.

Use of the hereinafter described and illustrated embodiment provides a better shaped and substantially increased lesion volume while working within the energy limits imposed by local tissue boiling, vaporization and charring. The use of multiple radially displaced umbrella electrode sets communicating current between them delivers more energy to the tumor without necessarily increasing the total amount of energy delivered. The voltage may be an oscillating voltage waveform having substantial energy in the spectrum below 500 kHz and preferably below 100 kHz.

The present inventors have further recognized that the impedance of tumor tissue differs significantly from that of regular tissue at frequencies below 100 kHz and especially below 10 kHz. This discovery can be exploited to preferentially ablate tumor tissue by proper selection of the frequency of the electrical energy.

The temperature at the first or second electrode sets can be monitored and the voltage delivered to the electrodes controlled as a function of that temperature.

It is thus possible to employ temperature feedback systems of the prior art with the present invention to increase, to the extent possible, the total energy delivered by the electrodes.

The first and second electrode sets may comprise umbrella electrodes. Each electrode set has at least three electrode wires extending radially from a support shaft to a radius from the support shaft. The first and second locations may be separated by an amount less than six times (and preferably four times) the maximum radius to which the electrode wires are extended.

The electrode sets may be separated by an amount that maximizes the useful size of the contiguous lesion volume.

Use of the electrode assembly may involve placing an additional conductor in contact to provide a diffuse return path for current (for example), a conductive plate against the skin of the patient.

Use of the electrode assembly may enable greater control over the current flow through the tumor, particularly in situations where inhomogeneities in the tissue would normally render one electrode much cooler than the other. Such inhomogeneities can include, for example, nearby blood vessels which carry heat away from nearby tissue. By using the conductive plate to augment current flow in one electrode, energy delivery at that electrode may be increased without changing the energy delivery at the other electrode.

Use of the electrode assembly may also include the step of placing at least one third electrode percutaneously at a third location different from the first and second locations but adjacent to the tumor and offset from the center of the tumor volume and monitoring the temperature at the first, second and third electrodes.

The electrode assembly may be part of a multi-electrode system that may define arbitrary volumes and accurately control temperature within those volumes for complete tumor ablation.

In the preferred embodiment of electrode assembly a support shaft has a shaft tip and a shank portion having a predetermined separation from the shaft tip, and the support shaft is sized for percutaneous placement of the shaft tip adjacent to the first location and the shank portion adjacent to the second location, with the first and second wire electrode sets being extensible radially from the support shaft at the first and second locations. A power supply is connectable between the first and second electrodes sets to induce a current flow there between.

A single shaft supporting the first and second wire sets in a predetermined separation corresponding to particular tissue characteristics and tumor sizes, simplifies use of the method. Multiple different shafts with different separations can be provided for different tumor sizes.

The ends of the electrode wire sets removed from the support shaft may be insulated.

It is an air with the electrode assembly of the invention to eliminate hot spots caused by high current densities at the tips of electrodes even in umbrella-type electrodes.

An insulating cover extends between the shaft and the shaft tip so as to prevent short circuit paths between the electrode sets through tissue and to the shaft.

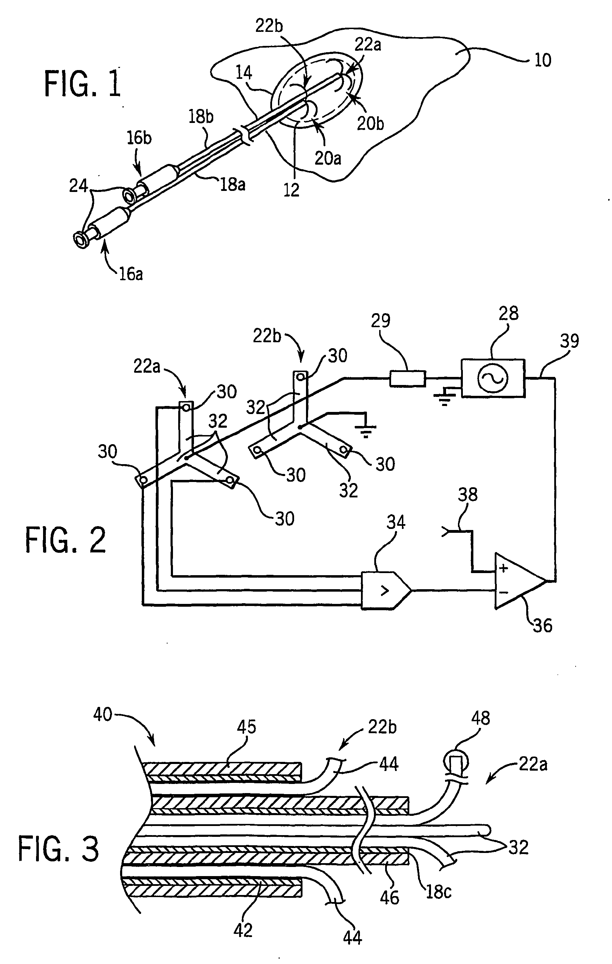

The foregoing and other features of the invention will appear from the following description. In this description, reference is made to the accompanying drawings, which form a part hereof, and in which there is shown by way of illustration, a preferred embodiment of the invention. Such embodiment and its advantages do not define the scope of the invention, however, and reference must be made therefore to the claims for interpreting the scope of the invention. The Fig.1 electrode assembly is not in accordance with the present invention due to the electrode sets being provided on separate shafts.

### BRIEF DESCRIPTION OF THE DRAWINGS

Fig. 1 is a perspective view of two umbrella electrode assemblies providing first and second electrode wires deployed at opposite edges of a tumor to create a lesion encompassing the tumor by a passing current between the electrodes;

Fig. 2 is a schematic representation of the electrodes of Fig. 1 as connected to a voltage controlled oscillator and showing temperature sensors on the electrode wires for feedback control of oscillator voltage;

Fig. 3 is a fragmentary cross-sectional view of a tip of a combined electrode assembly providing for the first and second electrode wires of Fig. 1 extending from a unitary shaft arranging the wires of the first and second electrodes in concentric tubes and showing an insulation of the entire outer surface of the tubes and of the tips of the electrode wires;

Fig. 4 is a simplified elevational cross-section of a tumor showing the first and second electrode positions and comparing the lesion volume obtained from two electrodes operating per the present invention, compared to the lesion volume obtained from two electrodes operating in a monopolar fashion;

Fig. 5 is a figure similar to that of Fig. 2 showing electrical connection of the electrodes of Fig. 3 to effect a more complex control strategy employing temperature sensing from each of the first and second electrodes and showing the use of a third skin contact plate held in voltage between the two electrodes so as to provide independent current control for each of the two electrodes;

Fig. 6 is a graph plotting resistivity in ohms-centimeters vs. frequency in Hz for tumorous and normal liver tissue, showing their separation in resistivity for frequencies below approximately 100 kHz;

Fig. 7 is a figure similar to that of Fig. 5 showing yet another embodiment in which wires of each of the first and second electrodes are electrically isolated so that independent voltages or currents or phases of either can be applied to each wire to precisely tailor the current flow between that wire and the other electrodes; and

Fig. 8 is a flow chart of a program as may be executed by the controller of Fig. 7 in utilizing its multi-electrode control.

### DETAILED DESCRIPTION OF THE INVENTION

Referring now to Fig. 1, a liver 10 may include a tumor 12 about which a lesion 14 can be created by using two umbrella-type electrode assemblies 16a and 16b having a slight modification as will be disclosed below. Each electrode assembly 16a and 16b has a thin tubular metallic shaft 18a and 18b sized to be inserted percutaneously into the liver 10. The shafts 18a and 18b terminate, respectively, at shaft tips 20a and 20b from which project trifurcated electrodes 22a and 22b which are formed of wires 32. The wires 32 are extended by means of a plunger 24 remaining outside the body once the shafts 18a and 18b are properly located within the liver 10 and when extended, project by an extension radius separated by substantially equal angles around the shaft tips 20a and 20b. The exposed ends of the wires 32 are preformed into arcuate form so that when they are extended from the shafts 18a and 18b they naturally splay outward in a radial fashion.

Umbrella electrode assemblies 16a and 16b of this type are well known in the art, but may be modified, by providing electrical insulation to all outer surfaces of the shafts 18a and 18b, in contrast to prior art umbrella electrode assemblies which leave the shaft tips 20a and 20b uninsulated, and by insulating the tips of the exposed portions of the wires 32. The purpose and effect of these modifications will be described further below.

The first electrode 22a is positioned at one edge of the tumor 12 and the other electrode 22b positioned opposite the first electrode 22a across the tumor 12 center. The term "edge" as used herein refers generally to locations near the periphery of the tumor 12 and is not intended to be limited to positions either in or out of the tumor 12, whose boundaries in practice, may be irregular and not well known. Of significance is that a part of the tumor 12 is contained between the electrodes 22a and 22b.

Referring now to Figs. 1 and 2, electrode 22a may be attached to a voltage controlled power oscillator 28 of a type well known in the art providing a settable frequency of alternating current power whose voltage amplitude (or current output) is controlled by an external signal. The return of the power oscillator 28 is connected to electrodes 22b also designated as a ground reference. When energized, power oscillator 28 induces a voltage between electrodes 22a and 22b causing current flow therebetween.

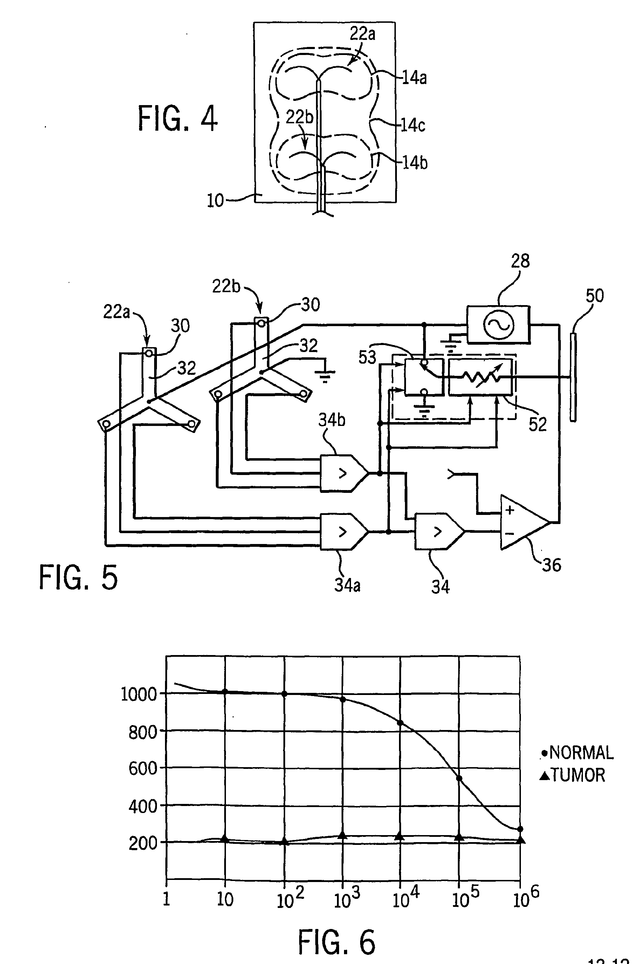

Referring now to Fig. 4, prior art operation of each electrode 22a and 22b being referenced to a skin contact plate (not shown) would be expected to produce lesions 14a and 14b, respectively, per the prior art. By connecting the electrodes as shown in Fig. 2, however, with current flow therebetween, a substantially larger lesion 14c is created. Lesion 14c also has improved symmetry along the axis of separation of the electrodes 22a and 22b. Generally, it has been found preferable that the electrodes 22a and 22b are separated by 2.5 to 3 cm for typical umbrella electrodes or by less than four times their extension radius.

Referring again to Fig. 2, temperature sensors 30, such as thermocouples, resistive or solid-state-type detectors, may be positioned at the distal ends of each of the exposed wires 32 of the tripartite electrodes 22a and 22b. For this purpose, the wires 32 may be small tubes holding small conductors and the temperature sensors 30 as described above. Commercially available umbrella-type electrode assemblies 16a and 16b currently include such sensors and wires connecting each sensor to a connector (not shown) in the plunger 24.

The temperature sensors 30 in electrode 22a may be connected to a maximum determining circuit 34 that selects the maximum output signal from the three temperature sensors of electrode 22. The maximum determining circuit 34 may be discrete circuitry, such as may provide precision rectifiers joined to pass only the largest signal, or may be implemented in software by first converting the signals from the temperature sensors 30 to digital values and determining the maximum by means of an executed program on a microcontroller or the like.

The maximum value of temperature from the temperature sensors 30 is passed by a comparator 36 (which also may be implemented in discrete circuitry or in software) which compares the maximum temperature to a predetermined desired temperature signal 38 such as may come from a potentiometer or the like. The desired temperature signal is typically set just below the point at which tissue boiling, vaporization or charring will occur.

The output from the comparator 36 may be amplified and filtered according to well known control techniques to provide an amplitude input 39 to the power oscillator 28. Thus it will be understood that the current between 22a and 22b will be limited to a point where the temperature at any one temperature sensors 30 approaches the predetermined desired temperature signal 38.

While the power oscillator 28 as described provides voltage amplitude control, it will be understood that current amplitude control may instead also be used. Accordingly, henceforth the terms voltage and current control as used herein should be considered interchangeable, being related by the impedance of the tissue between the electrodes 22b and 22a.

Alternatively current flowing between the electrodes 22a and 22b, measured as it flows from the power oscillator 28 through a current sensor 29, may be used as part of the feedback loop to limit current from the power oscillator 28 with or without the temperature control described above.

In yet a further possible arrangement not shown, the temperature sensors 30 of electrode 22b may also be provided to the maximum determining circuit 34 for more complete temperature monitoring. Other control methodologies may also be adopted including those provided for weighted averages of temperature readings or those anticipating temperature readings based on their trends according to techniques known to those of ordinary skill in the art.

Referring now to Fig. 3, the difficultly of positioning two separate electrode assemblies 16a and 16b per Fig. 1 may be reduced through the use of a unitary electrode 40 having a center tubular shaft 18c holding within its lumen, the wires 32 of first electrode 22a and a second concentric tubular shaft 42 positioned about shaft 18c and holding between its walls and shaft 18c wires 44 of the second electrode 22b. Wires 44 may be tempered and formed into a shape similar to that of wires 32 described above. Shafts 18c and 42 are typically metallic and thus are coated with insulating coatings 45 and 46, respectively, to ensure that any current flow is between the exposed wires 32 rather than the shafts 18c and 42.

As mentioned above, this insulating coating 46 is also applied to the tips of the shafts 18a and 18b of the electrode assemblies 16a and 16b of Fig. 1 to likewise ensure that current does not concentrate in a short circuit between the shafts 18a and 18b but in fact flows from the wires 32 of the wires of electrodes 22a and 22b.

Other similar shaft configurations for a unitary electrode 40 may be obtained including those having side-by-side shafts 18a and 18b attached by welding or the like.

Kits of unitary electrode 40 each having different separations between first electrode 22a and second electrode 22a may be offered suitable for different tumor sizes and different tissue types.

As mentioned briefly above, in either of the arrangements of Figs. 1 and 3 the wires 32 may include insulating coating 48 on their distal ends removed from shafts 18c and 42 so as to reduce high current densities associated with the ends of the wires 32.

In a preferred embodiment, the wires of the first and second electrodes 22a and 22b are angularly staggered (unlike as shown in Fig. 2) so that an axial view of the electrode assembly reveals equally spaced non-overlapping wires 32. Such a configuration is also desired in the arrangement of Fig. 2, although harder to maintain with two electrode assemblies 16a and 16b.

The frequency of the power oscillator 28 may be preferentially set to a value much below the 450 kHz value used in the prior art. Referring to Fig. 6, at less than 100 kHz and being most pronounced at frequencies below 10 kHz, the impedance of normal tissue increases to significantly greater than the impedance of tumor tissue. This difference in impedance is believed to be the result of differences in interstitial material between tumor and regular cell tissues although the present inventors do not wish to be bound by a particular theory. In any case, it is currently believed that the lower impedance of the tumorous tissue may be exploited to preferentially deposit energy in that tissue by setting the frequency of the power oscillator 28 at values near 10 kHz. Nevertheless, this frequency setting is not required in all embodiments of the present invention.

Importantly, although such frequencies may excite nerve tissue, such as the heart, such excitation is limited by the present bi-polar design.

Referring now to Fig. 5, the local environment of the electrodes 22a and 22b may differ by the presence of a blood vessel or the like in the vicinity of one electrode such as substantially reduces the heating of the lesion 14 in that area. Accordingly, it may be desired to increase the current density around one electrode 22a and 22b without changing the current density around the other electrode 22a and 22b. This may be accomplished by use of a skin contact plate 50 of a type used in the prior art yet employed in a different manner in the present invention. As used herein, the term contact plate 50 may refer generally to any large area conductor intended but not necessarily limited to contact over a broad area at the patient's skin.

In the embodiment of Fig. 5, the contact plate 50 may be referenced through a variable resistance (potentiometer) 52 to either of the output of power oscillator 28 or ground per switch 53 depending on the temperature of the electrodes 22a and 22b. Generally, switch 53 will connect the free end of variable resistance 52 to the output of the power oscillator 28 when the temperature sensors 30 indicate a higher temperature on electrode 22b than electrode 22a. Conversely, switch 53 will connect the free end of variable resistance 52 to ground when the temperature sensors 30 indicate a lower temperature on electrode 22b than electrode 22a. The comparison of the temperatures of the electrodes 22a and 22b may be done via maximum determining circuits 34a and 34b, similar to that described above with respect to Fig. 2. The switch 53 may be a comparator driven solid-state switch of a type well known in the art.

The output of the maximum determining circuits 34a and 34b each connected respectively to the temperature sensors 30 of electrodes 22a and 22b may also be used to control the setting of the variable resistance 52. When the switch 53 connects the variable resistance 52 to the output of the power oscillator 28, the maximum determining circuits 34a and 34b serve to reduce the resistance of variable resistance 52 as electrode 22b gets relatively hotter. Conversely, when the switch 53 connects the variable resistance 52 to ground, the maximum determining circuits 34a and 34b serve to reduce the resistance of variable resistance 52 as electrode 22a gets relatively hotter. The action of the switch 53 and variable resistance 52 is thus generally to try to equalize the temperature of the electrodes 22a and 22b.

If electrode 22a is close to a heat sink such as a blood vessel when electrode 22b is not, the temperature sensors 30 of electrode 22a will register a smaller value and thus the output of maximum determining circuit 34a will be lower than the output of maximum determining circuit 34b.

The variable resistance 52 may be implemented as a solid state devices according to techniques known in the art where the relative values of the outputs of maximum determining circuits 34a and 34b control the bias and hence resistance of a solid state device or a duty cycle modulation of a switching element or a current controlled voltage source providing the equalization described above.

Referring now to Fig. 7, these principles may be applied to a system in which each wire 32 of electrodes 22a and 22b is electrically isolated within the electrode assemblies 16a and 16b and driven by separate feeds 53 through variable resistances 54 connected either to the power oscillator 28 or its return. Electrically isolated means in this context that there is not a conductive path between the electrodes 22a and 22b except through tissue prior to connection to the power supply or control electronics. As noted before, a phase difference can also be employed between separate feeds 53 to further control the path of current flow between electrode wires 32. This phase difference could be created, e.g. by complex resistances that create a phase shift or by specialized waveform generators operating according to a computer program to produce an arbitrary switching pattern. The values of the resistances 54 are changed as will be described by a program operating on a controller 56. For this purpose, the variable resistances 54 may be implemented using solid-state devices such as MOSFET according to techniques known in the art.

Likewise, similar variable resistances 54 also controlled by a controller 56 may drive the contact plate 50.

For the purpose of control, the controller 56 may receive the inputs from the temperature sensors 30 (described above) of each wire 32 as lines 58. This separate control of the voltages on the wires 32 allows additional control of current flows throughout the tumor 12 to be responsive to heat sinking blood vessels or the like near any one wire.

Referring to Fig. 8, one possible control algorithm scans the temperature sensors 30 as shown by process block 60. For each temperature sensor 30, if the temperature at that wire 32 is above a "ceiling value" below a tissue charring point, then the voltage at that wire is reduced. This "hammering down" process is repeated until all temperatures of all wires are below the ceiling value.

Next at process block 62, the average temperature of the wires on each electrode 22a and 22b is determined and the voltage of the contact plate 50 is adjusted to incrementally equalize these average values. The voltage of the contact plate 50 is moved toward the voltage of the electrode 22 having the higher average.

Next at process block 64 the hammering down process of process block 60 is repeated to ensure that no wire has risen above its ceiling value.

Next at process block 66 one wire in sequence at each occurrence of process block 66 is examined and if its temperature is below a "floor value" below the ceiling value but sufficiently high to provide the desired power to the tumor, the voltage at that wire 32 is moved incrementally away from the voltage of the wires of the other electrode 22. Conversely, if the wire 32 is above the floor value, no action is taken.

Incrementally, each wire 32 will have its temperature adjusted to be within the floor and ceiling range by separate voltage control.

As shown in Fig. 7, this process may be extended to an arbitrary number of electrodes 22 including a third electrode set 22c whose connections are not shown for clarity.

While this present invention has been described with respect to umbrella probes, it will be understood that most of its principles can be exploited using probes extensible radially from a shaft to an extension radius and energized in a bipolar configuration. Further it will be understood that the present invention is not limited to two electrode sets, but may be used with multiple electrode sets where current flow is between first and second sets of the electrodes. The number of wires of the umbrella electrodes is likewise not limited to three and commercially available probes suitable for use with the present invention include a 10 wire version. Further although the maximum temperatures of the electrodes were used for control in the above-described examples, it will be understood that the invention is equally amenable to control strategies that use average temperature or that also evaluate minimum temperatures.

It is specifically intended that the present invention not be limited to the embodiments and illustrations contained herein, but modified forms of those embodiments including portions of the embodiments and combinations of elements of different embodiments as come within the scope of the following claims.

## Claims

1. An electrode assembly (40) for ablating tumors in a patient, the assembly comprising:
(a) a support shaft (18c) sized for percutaneous placement, said shaft having an outer surface and a distal tip;
(b) first and second wire electrode sets (22a, 22b) extensible radially from the shaft (18c) to an extension radius, the first wire electrode set (22a) being positionable at a first location adjacent to a tumour volume and offset axially along the support shaft from the second wire electrode set (22b), which second wire electrode set is positionable at a second location offset from the first location about the tumour volume, the first and second electrode sets each comprising at least three wires (32) positionable at angularly offset radial point around the support shaft;
(c) a power supply (28) connectable between the first and second electrode sets (22a, 22b) to induce a current flow between the first and second electrode sets whereby to concentrate current induced heating in the tumour volume;
wherein the support shaft has an electrically insulating cover (46) on the outer surface between the first and second locations, said cover extending to the distal tip of the support shaft.

2. The electrode assembly of claim 1, further comprising at least one temperature sensor (30) coupled to each of the first and second wire electrode sets (22a, 22b).

3. The electrode assembly of claim 2, further comprising control means (34, 34a, 34b, 36) connected to said temperature sensors (30) to receive temperature level signals from each of the first and second electrode sets (22a, 22b) and to control the voltage level applied to the first and second electrode sets as a function of the temperature level.

4. The electrode assembly as claim 3, wherein the electrode wires (32) in each of the first and second electrode sets (22a, 22b) arc electrically isolated, a said temperature sensor (30) is coupled to each of the wires in the electrode wire sets, and the control means monitors the temperature at each of the electrode wires and individually controls the voltage applied to the electrode wires.

5. The electrode assembly of any one of the preceding claims, wherein the electrode wires (32) in the first electrode set (22a) are axially aligned with the electrodes wires in the second electrode set (22).

6. The electrode assembly of any one of the preceding claims, wherein the electrode wires (32) of a respective electrode (22a, 22b) set are offset at substantially equal angles around the support shaft.

## Patentansprüche

1. Elektrodenanordnung (40) zum operativen Entfernen von Tumoren in einem Patienten, wobei die Anordnung umfasst:
(a) eine Unterstützungswelle (18c), die für eine perkutane Anordnung bemessen ist, wobei die Welle eine äußere Oberfläche und eine distale Spitze besitzt;
(b) eine erste und eine zweite Drahtelektrodengruppe (22a, 22b), die von der Welle (18c) bis zu einem Ausdehnungsradius radial ausgedehnt werden können, wobei die erste Drahtelektrodengruppe (22a) an einem ersten Ort benachbart zu einem Tumorvolumen und längs der Unterstützungswelle axial versetzt zu der zweiten Drahtelektrodengruppe (22b) positionierbar ist und wobei die zweite Drahtelektrodengruppe an einem zweiten Ort positionierbar ist, der zu dem ersten Ort um das Tumorvolumen versetzt ist, wobei die erste und die zweite Elektrodengruppe jeweils wenigstens drei Drähte (32) umfassen, die an in Winkelrichtung versetzten radialen Punkten um die Unterstützungswelle positionierbar sind; und
(c) eine Leistungsversorgung (28), die zwischen die erste und die zweite Elektrodengruppe (22a, 22b) geschaltet werden kann, um zwischen der ersten und der zweiten Elektrodengruppe einen Stromfluss zu induzieren, wodurch in dem Tumorvolumen durch Strom induzierte Wärme konzentriert wird;
wobei die Unterstützungswelle an der äußeren Oberfläche zwischen dem ersten und dem zweiten Ort eine elektrische isolierende Abdeckung (46) besitzt, wobei sich die Abdeckung zu der distalen Spitze der Unterstützungswelle erstreckt.

2. Elektrodenanordnung nach Anspruch 1, die ferner wenigstens einen Temperatursensor (30) umfasst, der sowohl mit der ersten als auch mit der zweiten Drahtelektrodengruppe (22a, 22b) gekoppelt ist.

3. Elektrodenanordnung nach Anspruch 2, die ferner Steuermittel (34, 34a, 34b, 36) umfasst, die mit den Temperatursensoren (30) verbunden sind, um Temperaturniveausignale von der ersten und der zweiten Elektrodengruppe (22a, 22b) zu empfangen und um den Spannungspegel, der an die erste und an die zweite Elektrodengruppe angelegt wird, als Funktion des Temperaturniveaus zu steuern.

4. Elektrodenanordnung nach Anspruch 3, wobei die Elektrodendrähte (32) sowohl in der ersten als auch in der zweiten Elektrodengruppe (22a, 22b) elektrisch isoliert sind, der Temperatursensor (30) mit jedem der Drähte in den Elektrodendrahtgruppen gekoppelt ist und die Steuermittel die Temperatur bei jedem der Elektrodendrähte überwachen und die an die Elektrodendrähte angelegte Spannung einzeln steuern.

5. Elektrodenanordnung nach einem der vorhergehenden Ansprüche, wobei die Elektrodendrähte (32) in der ersten Elektrodengruppe (22a) auf die Elektrodendrähte in der zweiten Elektrodengruppe (22) axial ausgerichtet sind.

6. Elektrodenanordnung nach einem der vorhergehenden Ansprüche, wobei die Elektrodendrähte (32) einer entsprechenden Elektrode (22a, 22b) um im Wesentlichen gleiche Winkel um die Unterstützungswelle versetzt sind.

## Revendications

1. Ensemble (40) d'électrodes pour l'ablation de tumeurs chez un patient, l'ensemble comprenant :
(a) une tige-support (18c) ayant des dimensions permettant une mise en place percutanée, ladite tige ayant une surface extérieure et une pointe distale ;
(b) un premier et un second jeux (22a, 22b) de fils-électrodes pouvant s'étendre radialement depuis la tige (18c) suivant un rayon d'extension, le premier jeu (22a) de fils-électodes pouvant être disposé à un premier endroit adjacent à un volume constituant une tumeur et décalé axialement le long de la tige-support par rapport au second jeu (22b) de fils-électrodes, lequel second jeu de fils-électrodes peut être disposé à un second endroit décalé par rapport au premier endroit autour du volume constituant une tumeur, les premier et second jeux d'électrodes comportant chacun au moins trois fils (32) pouvant être disposés en des points radiaux à décalage angulaire autour de la tige-support ;
(c) une source (28) d'électricité pouvant se brancher entre les premier et second jeux (22a, 22b) d'électrodes pour provoquer le passage d'un courant entre les premier et second jeux d'électrodes afin de concentrer de la sorte, dans le volume constituant une tumeur, le chauffage induit par le courant :
dans lequel la tige-support a, sur sa surface extérieure, une gaine électriquement isolante (46) entre les premier et second endroits, ladite gaine s'étendant jusqu'à la pointe distale de la tige-support.

2. Ensemble d'électrodes selon la revendication 1, comprenant en outre au moins un capteur (30) de température couplé à chacun des premier et second jeux (22a, 22b) de fils-électrodes.

3. Ensemble d'électrodes selon la revendication 2, comprenant en outre un moyen de commande (34, 34a, 34b, 36) connecté auxdits capteurs (30) de température pour recevoir de chacun des premier et second jeux (22a, 22b) d'électrodes des signaux de niveau de température et pour commander, en fonction du niveau de température, le niveau de la tension appliquée aux premier et second jeux d'électrodes.

4. Ensemble d'électrodes selon la revendication 3, dans lequel les fils-électrodes (32) de chacun des premier et second jeux (22a, 22b) d'électrodes sont isolés électriquement, un dit capteur (30) de température est couplé à chacun des fils des ensembles de fils-électrodes, et le moyen de commande contrôle la température sur chacun des fils-électrodes et commande individuellement la tension appliquée aux fils-électrodes.

5. Ensemble d'électrodes selon l'une quelconque des revendications précédentes, dans lequel les fils-électrodes (32) du premier jeu (22a) d'électrodes sont alignés axialement avec les fils-électrodes du second jeu (22b) d'électrodes.

6. Ensemble d'électrodes selon l'une quelconque des revendications précédentes, dans lequel les fils-électrodes (32) d'un jeu respectif (22a, 22b) d'électrodes sont décalés suivant des angles sensiblement égaux autour de la tige-support.
